# EUROPÄISCHE PATENTANMELDUNG

(11) **EP 2 138 205 A1**
(43) Veröffentlichungstag der Anmeldung: **30.12.2009**
(21) Anmeldenummer: 09160958.6
(22) Anmeldetag: 25.05.2009
(51) Int. Cl.: A61Q 5/12, A61K 8/86

(54) **Haarkonditionierende Zubereitung mit einer besonderen Sensorik**

(30) Priorität: 27.06.2008 DE 102008030135
(71) Anmelder: Beiersdorf AG, 20245 Hamburg (DE)
(72) Erfinder: Demitz, Michael, 22529, Hamburg (DE); Köhler, Manuela, 20144, Hamburg (DE); Saladin, Sandra, 20144, Hamburg (DE); Mahadeshwar, Anand, 22529, Hamburg (DE); Wendicke, Silke, 22303, Hamburg (DE)

(57) **Zusammenfassung**

Kosmetische Haarbehandlungszubereitung vorgesehen für das Ausspülen nach der Anwendung mit kationischen und/oder nichtionischen Tensiden enthaltend Polyethylenglycol mit einer molaren Masse von 3800000 bis 4200000 g/mol in einer Konzentration von 0,01 bis 0,25 Gew.-%, besonders bevorzugt von 0,05 bis 0,2 Gew.

## Beschreibung

Haarspülungen werden zur Pflege nach der Reinigung der Haare angewendet. Ein wichtiger Faktor ist die Sensorik der Spülung während des Auftragens auf das Haar. Der Konsument verbindet mit einer reichhaltigen Haarspülung eine sehr gute Pflegewirkung. Viele Haarspülungen zeigen zwar eine gute Leistung hinsichtlich der Pflegeeigenschaften, haben jedoch eine schlechte Sensorik, vor allem im nassen Haar.

"Gehaltvoll" im Sinne dieser Schrift wird wie folgt festgelegt: Es wird gemessen in welchem Ausmaß das Produkt als Schicht/Film zwischen der Haarsträhne und den Fingern wahrnehmbar ist. Hierbei wird eine 10er Skala verwendet bei der 10 den höchstmöglichen Gehalt darstellt.

"Gleitvermögen" im Sinne dieser Schrift bedeutet das Gleiten der Finger mit mittlerem/gleichbleibenden Druck an der Haarsträhne von oben nach unten. Hierbei wird eine 10er Skala verwendet bei der 10 das höchstmögliche Gleitvermögen darstellt.

"Entwirrbarkeit" im Sinne dieser Schrift ist die Messung wie leicht sich die Haare nach dem Ausspülen des Produkts mit dem Kamm entwirren lassen. Die Haare sind dann entwirrt, wenn der Kamm einmal komplett von oben nach unten durch die Haarsträhne geführt werden kann. Hierbei wird eine 10er Skala verwendet bei der 10 die höchstmögliche Entwirrbarkeit darstellt.

"Kämmbarkeit" im Sinne dieser Schrift ist die Messung wie leicht sich das Haar mit dem Kamm durchkämmen lässt, nachdem die Haare entwirrt wurden. Hierbei wird eine 10er Skala verwendet bei der 10 die höchstmögliche Kämmbarkeit darstellt.

"Haarstruktur" im Sinne dieser Schrift bedeutet die Beurteilung des Haares durch horizontales Rollen der Haare zwischen Daumen und Zeigefinger/ Mittelfinger einer Hand. Dies wird an verschiedenen Stellen der Strähne beginnend von oben nach unten geprüft. Hierbei wird eine 10er Skala verwendet bei der 10 die höchstmögliche Haarstruktur darstellt.

"Weich geschmeidig" im Sinne dieser Schrift bedeutet das sensorische Empfinden nach Applikation des Produktes im nassen und trockenen Haar durch einen Experten Panel.

DE 102006030091 A1 offenbart kosmetische waschaktive Zubereitungen zur Reinigung der Haut und/oder zur Haar- und Kopfhautreinigung, enthaltend nichtionische, hochmolekulare Polyethylenglykole, Tenside und kationische Pflegestoffe, **dadurch gekennzeichnet, dass** der Schaumparameter Cremigkeit/Reichhaltigkeit den Wert 8 bis 10 aufweist.

EP 473349 B1 offenbart haarkosmetische Zubereitungen mit einem Polyethylenglycol mit wenigstens 500000 g/mol mittlerem Molekulargewicht, Öl oder Fett und einem nichtionischen Tensid.

EP1570833 A1 offenbart emulsionsförmige Haarbehandlungszubereitungen mit Emulgator, Öl, Silikonöl und PEG mit einem MW von mindestens 10000.

EP 1771152 A1 offenbart haarkosmetische Zubereitungen mit einem wasserlöslichen nichtionischen Polymer des Ethylenoxides und nichtionische wasserlösliche Zelluloseether.

Im Stand der Technik fand sich kein Hinweis, wie die Gleitfähigkeit des nassen Haares verbessert werden konnte. Diese Gleitfähigkeit ist wichtig für das sensorische Empfinden des Verbrauchers während der Produktapplikation. Je gleitfähiger ein Produkt ist, desto pflegender wird es wahrgenommen. Hierbei wird eine 10er Skala verwendet bei der 10 die höchstmögliche Gleitfähigkeit darstellt.

Es hat sich überraschend gezeigt, dass eine kosmetische Haarbehandlungszubereitung vorgesehen für das Ausspülen nach der Anwendung mit kationischen und/oder nichtionischen Tensiden enthaltend Polyethylenglycol mit einer molaren Masse von 3800000 bis 4200000 g/mol in einer Konzentration von 0,01 bis 0,25 Gew.-%, besonders bevorzugt von 0,05 bis 0,2 Gew.-% zu einer deutlichen Verbesserung der Sensorik während der Applikation der haarkonditionierenden Zubereitung führt. Des Weiteren wurde beobachtet, dass sich die Haarstruktur im nassen und trockenen Haar verbessert. Entscheidend hierbei ist jedoch, dass der Gehalt an dem Polyethylenglykol den Wert 0,2 Gew.-% nicht überschreitet. Sonst erhält man ein fadenziehendes Produkt, welches vom Verbraucher als negativ empfunden wird.

Dabei ist von Vorteil, wenn zusätzlich 5 bis 12 Gew.-% Glycerin enthalten sind. Dadurch wird Haut und Haar durch die Anwendung Feuchtigkeit gespendet. Gleichzeitig kann die Zubereitung auch bei niedrigen Temperaturen gelagert werden. Weiter vorteilhaft ist es, wenn zusätzlich 2 bis 6 Gew.-% Cetylalkohol oder Cetylstearylalkohol, besonders bevorzugt 3 bis 4 Gew.-% Cetylalkohol oder Cetylstearylalkohol enthalten sind. Dadurch erhält die Zubereitung ein für den Anwender angenehmes Fließverhalten und gleichzeitig wird die reichhaltige Sensorik verbessert. Weiter von großem Vorteil ist es, wenn zusätzlich 0,001 bis 0,08 Gew.-% Natriumcitrat oder 0,6 bis 0,8 Gew.-% Lactat enthalten sind. Dadurch werden die Pflegeeigenschaften des Produktes verbessert. Weiter von großem Vorteil ist es, wenn die Zubereitung frei von Cellulosederivaten ist. Weiter von großem Vorteil ist es, wenn die Zubereitung nicht waschaktiv ist. Weiter von großem Vorteil ist es, wenn die Zubereitung frei von anionischen Tensiden ist. Die Erfindung umfasst auch die Verwendung von Polyethylenglycol mit einer molaren Masse von 3800000 bis 4200000 g/mol in einer Konzentration von 0,01 bis 0,25 Gew.-% in einer kosmetischen Haarbehandlungszubereitung vorgesehen für das Ausspülen nach der Anwendung mit kationischen und/oder nichtionischen Tensiden, insbesondere einer Zubereitung nach einem der vorangehenden Patentansprüche zur Verbesserung des Gleitvermögens im nassen Haar.

Erfindungsgemäße Zubereitungen können zusätzlich UV- Filter, Polyole, Konservierungsmittel, hydrolysierte Proteine, Pflanzenextrakte, Parfümöle, und Antioxidantien enthalten.

### Beispiele

Von einem Expertenteam (n= 3) wurden zunächst 2 haarkonditionierende Formeln (Formeln 1,2 Tab .1) sensorisch bewertet. Die Formeln 1-3 unterscheiden sich lediglich im Gehalt des polymeren Ethylenoxids PEG-90M. Es wurde gefunden, dass Formel 2 während des Auftragens deutlich gehaltvoller ist als Formel 1 und die Formel 2 gegenüber Formel 1 einen positiveren Einfluss auf die Haarstruktur hat. Diese Ergebnisse zugunsten der Formel 2 konnten von dem Expertenteam auch im trockenen Haar bestätigt werden.

**Tabelle 1:**

| | wt % | 1 | 2 | 3 |
|---|---|---|---|---|
| 1 | Methylparaben | 0,25 | 0,25 | 0,25 |
| 2 | Propylparaben | 0,1 | 0,1 | 0,1 |
| 3 | Aqua | 83,99 | 83,94 | 83,24 |
| 4 | Cetyl Alcohol | 2,5 | 2,5 | 2,5 |
| 5 | Lactic Acid | 0,8 | 0,8 | 0,8 |
| 6 | Sodium Chloride | 0,01 | 0,01 | 0,01 |
| 7 | Stearyl Alcohol | 4,8 | 4,8 | 4,8 |
| 8 | Oryzanol | 0,05 | 0,05 | 0,05 |
| 9 | PEG-90 M | | 0,05 | 0,1 |
| 10 | Stearamidopropyl Dimethylamine | 2,2 | 2,2 | 2,2 |
| 11 | Parfum | 0,7 | 0,7 | 0,7 |
| 12 | Dimethicone | 4,6 | 4,6 | 4,4 |

Der positive Effekt bei Zugabe von PEG-90 M zu einer Haarspülung ist auch in einem Halbseitentest mit Probanden (n = 10) mit trocken/strapaziertem Haar erkennbar. Die Formel 2 ist der Formel 1 in allen Parametern überlegen (Tabelle 2).

**Tabelle 2:**

| | 1 | 2 |
|---|---|---|
| Gehalt verteilen | 7,50 | 7,80 |
| Gehalt sofort | 6,30 | 6,60 |
| Gleitvermögen s | 6,90 | 7,30 |
| Gleitvermögen 40s | 6,20 | 6,70 |
| Entwirrbarkeit n | 7,00 | 7,30 |
| Kämmbarkeit n | 7,30 | 7,70 |
| Haarstruktur n Längen | 6,90 | 7,60 |
| Haarstruktur n Spitzen | 5,80 | 6,10 |
| Gleitvermögen n | 6,70 | 7,20 |
| Entwirrbarkeit tr | 7,10 | 7,30 |
| Kämmbarkeit tr | 7,40 | 7,50 |
| Gleitfähigkeit tr | 6,90 | 7,10 |
| Struktur tr Längen | 7,20 | 7,30 |
| Struktur tr Spitzen | 6,20 | 6,50 |
| weich geschmeidig tr | 2,00 | 3,00 |

Die Verbesserung der Sensorik bei Zusatz von PEG-90 M lässt sich auch auf andere Pflege-Systeme übertragen. Nachfolgend sind Beispiele aufgeführt, in denen das Expertenteam ebenfalls einen positiven Einfluss des PEG-90Ms feststellen konnte. Der Einsatz von mehr als 0.2 % PEG-90 M wurde als negativ bewertet.

### Weitere erfindungsgemäße Zubereitungen:

| | wt% | 4 | 5 | 6 | 7 |
|---|---|---|---|---|---|
| 1 | Methylparaben | 0,25 | 0,25 | 0,25 | 0,25 |
| 2 | Propylparaben | 0,1 | 0,1 | 0,1 | |
| 3 | Aqua | 79,5 | 79 | 79,6 | 77,28 |
| 4 | Citric Acid | 0,025 | 0,025 | 0,025 | 0,01 |
| 5 | Glycerin | 10 | 10 | 10 | |
| 6 | Cetearyl Alcohol | 3 | 3,5 | 3,5 | 4 |
| 7 | Cetearyl Isononanoate | | | 0,5 | |
| 8 | Bis-Diglyceryl Polyacyladipate-2 | 1 | 1 | 1 | |
| 9 | Glycerin | | | | 8,7 |
| 10 | Sodium Hydroxide | 0,025 | 0,025 | 0,025 | 0,01 |
| 12 | Cetrimonium Chloride | 3 | 3 | 1 | |
| 13 | Polyglyceryl-3 Methylglucose Distearate | 0,5 | 0,5 | 0,5 | |
| 14 | Distearoylethyl Hydroxyethylmonium Methosulfate | 2 | 2 | 3 | |
| 15 | Oryzanol | | | | 0,05 |
| 16 | Isodecyl Neopentanoate | | | | 3 |
| 17 | PEG/PPG-17/18 Dimethicone | | | | 3 |
| 18 | PEG-90 M | 0,2 | 0,2 | 0,1 | 0,1 |
| 19 | Palmitamidopropyltrimonium Chloride | | | | 3 |
| 20 | Parfum | 0,4 | 0,4 | 0,4 | 0,6 |

| | wt % | 8 | 9 | 10 | 11 | 12 |
|---|---|---|---|---|---|---|
| 1 | Methylparaben | 0,25 | 0,25 | 0,25 | 0,25 | 0,25 |
| 2 | Propylparaben | 0,1 | 0,1 | 0,1 | 0,1 | 0,1 |
| 3 | Aqua | 80,98 | 87,555 | 80,225 | 85,325 | 78,355 |
| 4 | Octyldodecanol | | | | 0,5 | |
| 5 | Citric Acid | 0,01 | | 0,15 | | 0,005 |
| 6 | Cetearyl Alcohol | 3,15 | | 5,9 | | |
| 7 | Cetyl Alcohol | | 2 | | 2,5 | 2 |
| 8 | Dimethicone | | | | | 1 |
| 9 | Panthenol | | 0,15 | | | |
| 10 | Lactic Acid | | 0,8 | | 0,8 | |
| 11 | Benzophenone-4 | | | 0,25 | | 0,25 |
| 12 | Sodium Hydroxide | 0,01 | | 0,15 | | 0,06 |
| 13 | Sodium Chloride | | 0,01 | | 0,01 | |
| 14 | Hydrolyzed Silk | | 0,1 | | | |
| 15 | Stearyl Alcohol | | 3,8 | | 4,8 | 4 |
| 16 | Dicaprylyl Ether | | | | 0,5 | |
| 17 | Distearoylethyl Hydroxyethylmonium Methosulfate | | | 0,715 | | 2,86 |
| 18 | Oryzanol | 0,05 | 0,05 | 0,05 | 0,05 | 0,05 |
| 19 | Dimethicone | | | 1,5 | | |
| 20 | PEG-90 M | 0,05 | 0,05 | 0,05 | 0,05 | 0,05 |
| 21 | Cyclomethicone | | | | | 2 |
| 22 | Palmitamidopropyltrimonium Chloride | 2 | 0,415 | 1,66 | 0,415 | 3,32 |
| 23 | Hydroxypropyl Starch Phosphate | 2 | | | | |
| 24 | PPG-3 Benzyl Ether Myristate | 2 | | | 2 | |
| 25 | Stearamidopropyl Dimethylamine | | 2 | | 2 | |
| 26 | Parfum | 0,7 | 0,7 | 0,7 | 0,7 | 0,7 |
| 27 | Behentrimonium Methosulfate | | | 1 | | |
| 28 | Dimethicone | | | 2,3 | | |

## Patentansprüche

1. Kosmetische Haarbehandlungszubereitung vorgesehen für das Ausspülen nach der Anwendung mit kationischen und/oder nichtionischen Tensiden enthaltend Polyethylenglycol mit einer molaren Masse von 3800000 bis 4200000 g/mol in einer Konzentration von 0,01 bis 0,25 Gew.-%, besonders bevorzugt von 0,05 bis 0,2 Gew.-%.

2. Zubereitung nach Patentanspruch 1 **dadurch gekennzeichnet, dass** zusätzlich 5 bis 12 Gew.-% Glycerin enthalten sind.

3. Zubereitung nach einem der vorangehenden Patentansprüche **dadurch gekennzeichnet, dass** zusätzlich 2 bis 6 Gew.-% Cetylalkohol oder Cetylstearylalkohol, besonders bevorzugt 3 bis 4 Gew.-% Cetylalkohol oder Cetylstearylalkohol enthalten sind.

4. Zubereitung nach einem der vorangehenden Patentansprüche **dadurch gekennzeichnet, dass** zusätzlich 0,001 bis 0,08 Gew.-% Natriumcitrat oder 0,6 bis 0,8 Gew.-% Lactat enthalten sind.

5. Zubereitung nach einem der vorangehenden Patentansprüche **dadurch gekennzeichnet, dass** die Zubereitung frei von Cellulosederivaten ist.

6. Zubereitung nach einem der vorangehenden Patentansprüche **dadurch gekennzeichnet, dass** die Zubereitung nicht waschaktiv ist.

7. Zubereitung nach einem der vorangehenden Patentansprüche **dadurch gekennzeichnet, dass** die Zubereitung frei von anionischen Tensiden ist.

8. Verwendung von Polyethylenglycol mit einer molaren Masse von 3800000 bis 4200000 g/mol in einer Konzentration von 0,01 bis 0,25 Gew.-% in einer kosmetischen Haarbehandlungszubereitung vorgesehen für das Ausspülen nach der Anwendung mit kationischen und/oder nichtionischen Tensiden, insbesondere einer Zubereitung nach einem der vorangehenden Patentansprüche zur Verbesserung des Gleitvermögens im nassen Haar.
